Europäisches Patentamt

**European Patent Office**

**Office européen des brevets**

(11) Veröffentlichungsnummer: **0 290 032 B1**

# EUROPÄISCHE PATENTSCHRIFT

(45) Veröffentlichungstag der Patentschrift: **24.06.92**

(51) Int. Cl.⁵: **B65D 85/672**, A61F 15/00

(21) Anmeldenummer: **88107274.8**

(22) Anmeldetag: **06.05.88**

(54) **Spenderbox zur Entnahme insbesondere vorproportionierter Teilstücke von ein- oder mehrbahnig zu einer Rolle aufgewickeltem, bandförmigem Packungsgut, wie Verbandstoffe und/oder Verbandmaterial.**

(30) Priorität: **08.05.87 DE 8706604 U**

(43) Veröffentlichungstag der Anmeldung:
**09.11.88 Patentblatt 88/45**

(45) Bekanntmachung des Hinweises auf die
Patenterteilung:
**24.06.92 Patentblatt 92/26**

(84) Benannte Vertragsstaaten:
**AT BE DE FR NL**

(56) Entgegenhaltungen:
**CH-A- 435 550**
**US-A- 1 946 105**

(73) Patentinhaber: **Paul Hartmann Aktiengesellschaft**
**Paul-Hartmann-Strasse**
**W-7920 Heidenheim(DE)**

(72) Erfinder: **Zanker, Helmut**
**Neuestrasse 189 A**
**W-7000 Stuttgart 1(DE)**

(74) Vertreter: **Becker, Maria, Dipl.-Phys.**
**Auf dem Haigst 29**
**W-7000 Stuttgart 70(DE)**

Rank Xerox (UK) Business Services

## Beschreibung

Die Erfindung betrifft eine Spenderbox zur Entnahme insbesondere vorproportionierter Teilstücke von ein-oder mehrbahnig zu einer Rolle zugewikkeltem, bandförmigen Packungsgut, wie Verbandstoffe und/oder Verbandsmaterial, mit einem dieses aufnehmenden, verschliessbaren, quaderförmigen Gehäuse mit einer an dessen einer Gehäuseschmalseite vorgesehenen, mittels einer um eine Schwenkachse verschwenkbaren Verschlussklappe verschliessbaren, eine der Bahnbreite des Packungsgutes entsprechenden Breite aufweisenden schlitzförmigen Entnähmeöffnung.

Eine Spenderbox dieser Art ist bereits seit Jahren auf dem Markt (Siehe Dokument CH-A-435 550). Die Entnahmeöffnung ist in diesem Falle durch eine in der einen Gehäuseschmalseite vorgesehene, rechteckförmige Ausnehmung gebildet, deren Höhe einem Vielfachen der Höhe des bandförmigen Packungsmaterials entspricht und dit mittels einer am Gehäuse um eine an der Ausnehmung unten liegend angeordnete Schwenkachse verschwenkbaren Verschlussklappe staubdicht verschliessbar ist.

Hierzu ist die Verschlussklappe manuell nach oben zu verschwenken, was jedoch unter Praxisbedingungen keinesfalls immer sicherzustellen ist. In offenem Zustand der Entnahmeöffnung ragt die Verschlussklappe von der betreffenden Gehäusewand nach vorne ab und ist einem sicheren Zugriff des Packungsgutes insofern hinderlich, als dessen Ende auf der Innenseite der schalenförmigen Verschlussklappe aufliegt.

Die Erfindung zielt nun darauf ab, eine Spenderpackung mit den eingangs erläuterten Merkmalen so zu verbessern, dass die Verschlussklappe in ihrer Offenstellung einem sicheren Zugriff des aus der Entnahmeöffnung herauszuziehenden vorderen Endstückes des Packungsgutes nicht mehr im Wege steht und nach jeder Entnahme eines Teilstückes vom Packungsgut ein zuverlässig staubdichter Verschluss der Entnahmeöffnung sichergestellt ist.

Diese Aufgabe wird erfindungsgemäss durch die kennzeichnenden Merkmale des Anspruches 1 gelöst.

Bei der erfindungsgemässen Konstruktion wird allein schon aufgrund der entsprechend verengten Ausbildung der Entnahmeöffnung ein Eindringen von Staubpartikeln in den das ausgerollte Packungsgut aufnehmenden Gehäuseraum bei geöffneter Entnahmeöffnung beträchtlich erschwert und eine definierte Führung des Packungsgutes erreicht. Nach Entnahme entsprechender Teilstücke des Packungsgutes bewegt sich die Verschlussklappe von selbst in ihre Schliesstellung, wobei die das Auflager für sie bildende Vorderkante der einen Schlitzlängswand einen im hohen Maße sicheren Staubverschluss gewährleistet.

Die Verschlussklappe kann sich hierbei unter Schwerkrafteinfluss oder mittels einer Rückstellfeder in ihre Schliesstellung bewegen, wobei deren Anordnung oderhalb der unteren Schlitzlängswand bzw. des Auflagers eine vorteilhafte Freigabe der Entnahmeöffnung ermöglicht, indem sie beispielsweise mit einem Zeigefinger nach oben geklappt und mit diesem und dem Daumen das aus der Entnahmeöffnung herauszuziehende Endstück des Packungsgutes zugleich problemlos erfasst und aus dem Gehäuse herausgezogen werden kann.

Die Entnahmeöffnung kann hierbei an einer der Gehäuseschmalseiten oben, bevorzugt jedoch unten, vorgesehen sein. Für eine vorteilhafte Handhabung der Verschlussklappe sowie einer weiteren Verbesserung des Staubverschlusses der Entnahmeöffnung ist eine Ausführungsform nach Anspruch 2 günstig. Dabei lässt sich das Auflager gemäss den Ansprüchen 3 und 4 zugleich als Abrisskante benutzen, so dass sich vom Packungsgut insbesondere vorproportionierte Teilstücke durch einfache Zugbeanspruchung des Packungsgutes in Längsrichtung von diesem am Auflager abreissen lassen.

Eine Konstruktion gemäss den Ansprüchen 5 und 6 bietet den Vorteil, dass nach Abtrennen wenigstens eines Teilstückes vom Packungsgut dessen Endstück griffbereit auf dem unteren, über die Vorderkante des oberen Wandteils vorstehenden Wandteils verbleibt und nach Abheben der Verschlussklappe vom stegartigen Auflager dieses Wandteils besonders vorteilhaft ergriffen werden kann.

Eine Anordnung der Verschlussklappe nach Anspruch 7 bildet den Vorteil, an dieser gemäss Anspruch 8 einen Steg anformen zu können, der in der Schliesstellung der Verschlussklappe zumindest auf dem Packungsgut aufruht und somit eine zusätzliche Staubsperre bildet.

Dieser Steg kann beispielsweise einen halbkreisförmigen, ovalen oder auch keilförmigen Querschnitt aufweisen. In einer Ausgestaltung dieses Steges in Anspruch 9 legt sich dieser in der Schliesstellung der Verschlussklappe unter Druck auf das dem unteren Wandteil aufruhende Endstück des Packungsgutes auf, was bewirkt, dass es dort festgelegt wird, und somit bei einem Ortswechsel der Spenderbox in seiner Entnahmeposition verbleibt und nicht in das Gehäuse zurückgleiten kann. Ausserdem vermag der Steg in diesem Falle auch Transportfunktion zu übernehmen, indem dieser beim Hochschwenken der Verschlussklappe das Endstück des Packungsgutes in Ausziehrichtung verschiebt.

Weitere Merkmale und Einzelheiten der Erfindung sind Gegenstand weiterer Unteransprüche.

In der Zeichnung sind Ausführungsbeispiele der Erfindung dargestellt. Es zeigen:

Fig. 1     eine teilweise aufgebrochene Seitenansicht einer ersten Ausführungsform einer Spenderbox;

Fig. 2     eine Stirnansicht der Spenderbox, in Richtung des Pfeiles A der Fig. 1 gesehen;

Fig. 3     einen Schnitt entlang der Linie 3-3 der Fig. 1;

Fig. 3a     eine Konstruktionsvariante bezüglich der Anordnung der Verschlussklappe der Spenderbox;

Fig. 4     einen Längsschnitt durch eine zweite Ausführungsform einer Spenderbox;

Fig. 5     einen Querschnitt der Spenderbox entlang der Linie 5-5 der Fig. 4;

Fig. 6     einen in Fig. 4 durch einen strichpunktierten Kreis angedeuteten Ausschnitt im vergrösserten Maßstab.

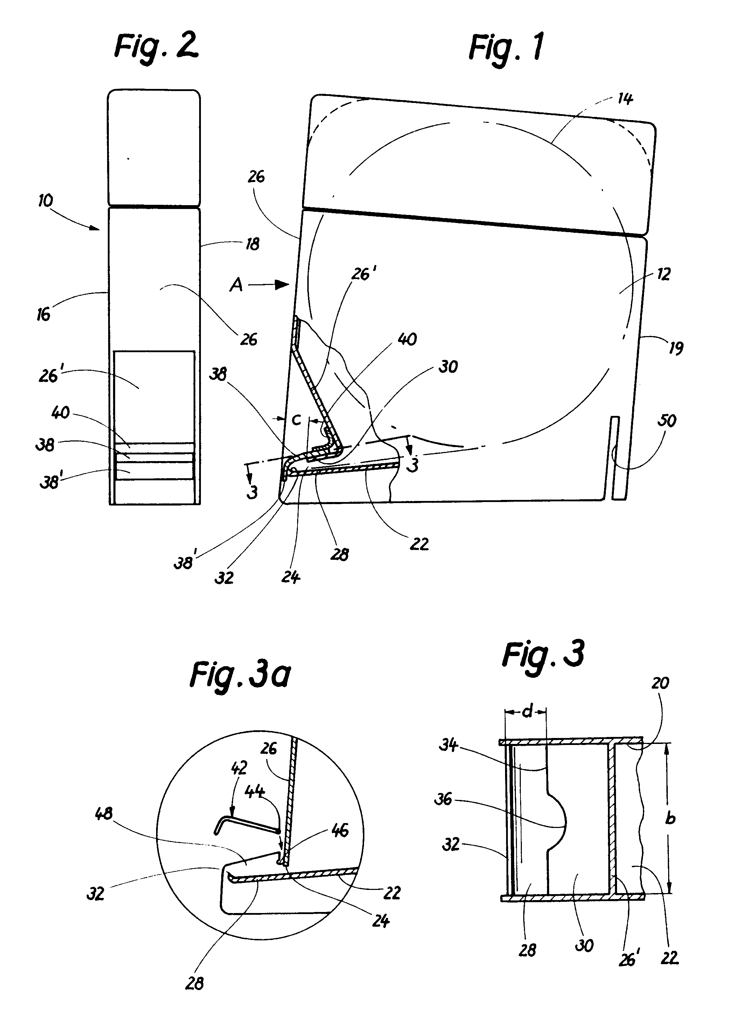

Die in den Fig. 1 und 2 gezeigte Spenderbox weist ein Gehäuse 10 auf, dessen oben offenes Gehäuseunterteil 12 mittels eines auf diesen aufsetzbaren Gehäusedeckels 14 verschliessbar ist. Der zwei zueinander parallele ebene Gehäuseseitenwände 16, 18 aufweisende Gehäuseunterteil 12 weist gemäss Fig. 3 einen Gehäuseinnenraum 20 auf, dessen Breite b geringfügig grösser ist als die Breite einer aus aufgewickeltem Packungsgut bestehenden Vorratsrolle, die in das Gehäuse einzusetzen ist und auf dessen Gehäuseboden 22 aufruht. Bei diesem Packungsgut kann es sich beispielsweise um eine bandförmige, längs von Perforationslinien in Zellstofftupfer zertrennbare Zellstoffbahn handeln.

Mit 24 ist eine schlitzförmige Entnahmeöffnung bezeichnet, die sich im Bereich des Gehäusebodens 22 an der mit 26 bezeichneten vorderen Gehäusestirnwand befindet. Diese erstreckt sich über die gesamte Breite der letzteren. Sie ist definiert durch einen unteren Wandteil 28 und einen oberen Wandteil 30, die einander im wesentlichen parallel zugeordnet sind und deren gegenseitiger Abstand geringfügig grösser ist als die Dicke des aus der Entnahmeöffnung 24 herauszuziehenden Packungsgutes.

Wie Fig. 1 zeigt, endet die Vorderkante des oberen Wandteils 30 in einem Abstand c von der vorderen Gehäusestirnwand 26. Zu diesem Zweck bildet der obere Wandteil 30 ein nach vorne gerichtetes Randstück eines unteren Teilstückes 26' der vorderen Gehäusestirnwand 26, das zu diesem Zweck zwischen den Gehäuseseitenwänden 16 und 18 schräg nach unten und hinten verläuft. Der untere Wandteil 28 bildet eine Verlängerung des in Richtung der schlitzförmigen Entnahmeöffnung 24 vorzugsweise schräg abfallenden Gehäusebodens 22 und erstreckt sich im wesentlichen bis in die Ebene der vorderen Gehäusestirnwand 26.

Wie insbesondere aus Fig. 3a ersichtlich ist, bildet das vordere Randstück des unteren Wandteils 28 einen nach oben gerichteten und in dieser Richtung sich vorzugsweise keilförmig verjüngenden Auflagesteg 32, der aufgrund seiner relativ scharfkantigen Ausbildung als Abreisskante benutzt werden kann.

Wie aus Fig. 3 ersichtlich ist, verbleibt vor der Entnahmeöffnung 24 über einen Bereich d ein vom oberen Wandteil 30 nicht abgedeckter Bereich des unteren Wandteils 28, auf welchem das nach Abreissen eines Teilstückes von Packungsgut verbleibende Endstück aufliegen bleibt. Um dieses ohne Schwierigkeiten ergreifen zu können ist in der Längsmitte der oberen Wandteils 30 eine von dessen Vorderkante 34 aus eingearbeitete Aussparung 36 in Daumenbreite vorgesehen.

Mit 38 ist eine Verschlussklappe bezeichnet, die mit ihrem hinteren Randstück den oberen Wandteil 30 übergreift und mittels eines Scharnierbandes 40 am unteren Teilstück 26' der Gehäusestirnwand 26 angelenkt ist. Diese Verschlussklappe 38 überdeckt den zwischen der Vorderkante 34 des oberen Wandteils 30 und dem Auflagesteg 32 über deckungsfreien, sich an die Entnahmeöffnung 24 anschliessenden Bereich und liegt in ihrer Schliessstellung auf dem durchgehenden Auflagesteg 32 auf und schafft damit einen in hohem Maße staubdichten Verschluss im Bereich der Entnahmeöffnung 24. Vorzugsweise übergreift dabei die Verschlussklappe 38 in der Schliesstellung den Auflagesteg 32 mit seinem vorderen Klappenrandstück 38'.

Zum Herausziehen und Abreissen eines Teilstückes von Packungsgut ist die Verschlussklappe 38 und die durch das Scharnierband 40 definierte Schwenkachse gemäss Fig. 1 nach oben zu klappen, wonach das im überdeckungsfreien Bereich d (Fig. 3) vorhandene Endstück des Packungsgutes ergriffen, über den Auflagesteg 32 weggezogen und insbesondere längs einer Perforationslinie und unter entsprechender Zugbelastung entlang des Auflagesteges 32 abgerissen werden kann. Hierbei ruht die Verschlussklappe 38 mit ihrem vorderen Klappenrandstück 38' auf der Oberseite des herauszuziehenden Teilstückes des Packungsgutes auf und legt sich nach Abreissen des Teilstückes selbsttätig wieder auf den Auflagestag 32 zum staubdichten Verschliessen der Entnahmeöffnung auf.

Bei der in Fig. 3a gezeigten Konstruktionsvariante ist die Entnahmeöffnung 24 durch das untere Randstück der vorderen Gehäusestirnwand 26 gebildet und eine mit 42 bezeichnete Verschlussklappe ist mit seitlichen Lagerzapfen 44 in entsprechend Lagerausnehmungen 46 schwenkgeweglich einhängbar, die in sich über die vordere Gehäusestirnwand 26 hinaus erstreckenden unteren Wandansatzstücken 48 eingeformt sind, zwischen denen sich auch der untere Wandteil 28 des Gehäusebodens 22 befindet.

Wie aus Fig. 1 und 2 ersichtlich ist, stehen die Gehäuseseitenwände 16, 18 sowie die hintere Gehäuserückwand 19 über den Gehäuseboden nach unten vor. In den beiden Gehäuseseitenwänden 16, 18 und ggf. auch in der Gehäuserückwand 19 ist jeweils eine sich von unten nach oben erstreckende, insbesondere schlitzförmige Ausnehmung 50 eingebracht, die es ermöglicht, die Spenderbox an Schienen oder anderen geeigneten Halteeinrichtungen aufnehmungen zu können. Hierbei können bei Bedarf eine oder mehrere Spenderboxen auf einer solchwn Halteeinrichtung in kompakter oder aufgelockerter Form zusammengestellt werden.

Die Gehäuseseitenwände 16, 18 können im unteren Bereich rechtwinklig ausgebildet sein oder mit der vorderen Gehäusestirnwand 26, wie in Fig. 1 gezeigt, einen spitzen oder ggf. auch einen stumpfen Winkel bilden. Die dargestellte Ausführungsform mit spitzem Winkel erlaubt eine nach rückwärts beneigte Schrägstellung der Spenderbox, um eine leichtere, manuelle Entnahme zu ermöglichen. Eine Ausführungsform mit stumpfer Winkelanordnung ermöglicht eine Integration in Halterungssysteme in Standform oder für Hängemontage, wobei durch die Neigung der unteren Kanten der Gehäuseseitenwände 16, 18 eine sichere Positionierung auch in nicht rechtwinkligen Systemen mit besserer Entnahmemöglichkeit gegeben ist.

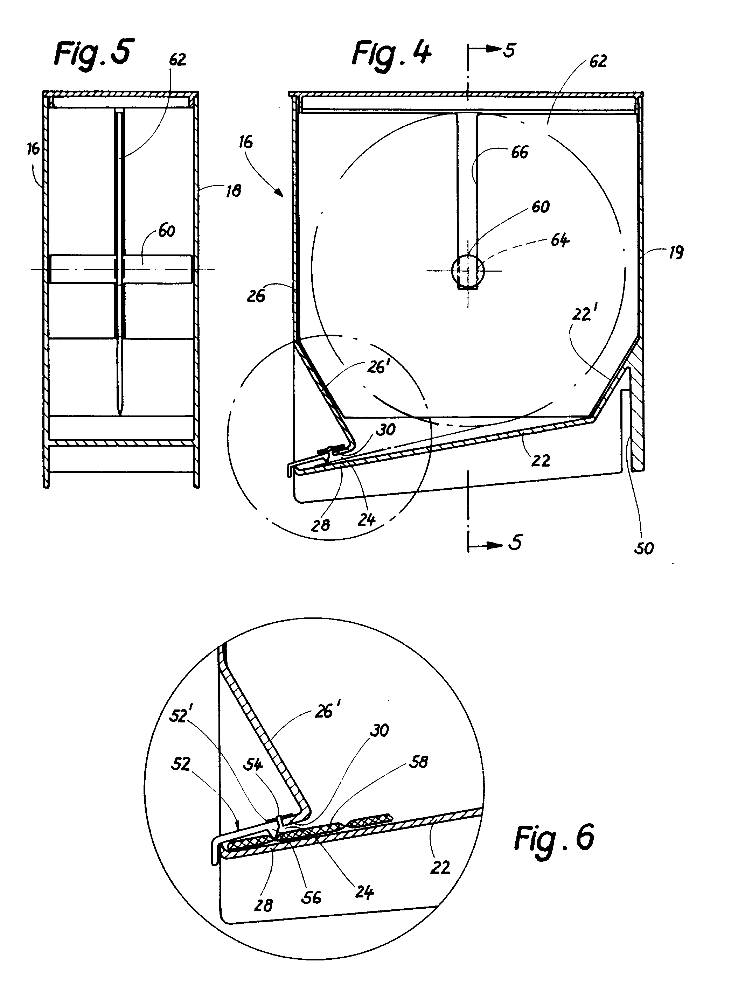

Beim Ausführungsbeispiel gemäss den Fig. 4 bis 6 ist der zusammen mit dem unteren Wandteil 28 die schlitzförmige Entnahmeöffnung 24 definierende obere Wandteil 30 verhältnismässig kurz ausgebildet und die als Ganzes mit 52 bezeichnete Verschlussklappe bildet in ihrer Schliesstellung gewissermassen eine Verlängerung des oberen Wandteils 30. 54 bezeichnet ein Scharnier zur schwenkbeweglichen Verbindung vom oberen Wandteil 30 und Verschlussklappe 52. Wie dabei aus Fig. 6 deutlich zu ersehen ist, ist die Verschlussklappe 52 an der Unterseite längs ihres den oberen Wandteil 30 benachbarten Randstückes 52' mit einem zur Vorderkante des oberen Wandteils 30 parallel verlaufenden Steg 56 ausgestattet, der im Querschnitt keilförmig und in seinem Kantenbereich vorzugsweise abgerundet ausgebildet ist und der in der gezeigten Schliesstellung der Verschlussklappe 52 auf dem mit 58 bezeichneten Packungsgut aufruht und dieses zwischen sich und dem unteren Wandteil 28 fixiert. Durch dieses Klemmen wird eine zusätzliche Abdichtung der Entnahmeöffnung 24 geschaffen sowie erreicht, dass bei einem Ortswechsel der Spenderbox das Packungsgut 58 in seiner Entnahmeposition verbleibt und nicht in den Gehäuseinnenraum 20 zurückgleiten wird.

Um das Abrollen schwererer Rollen von Packungsgut im Gehäuseinnenraum 20 zu erleichtern, weist der Gehäuseboden 22 ein rückwärtiges, schräg nach oben und hinten ansteigenden Bodenteilstück 22' auf, das in die Gehäuserückwand 19 übergeht. Desweiteren kann zu diesem Zweck eine massiv oder hohl ausgebildete Lagerwelle 60 vorgesehen sein, die mit ihren Enden entweder in entsprechenden Lageraufnahmen an den Innenseiten der Gehäuseseitenwände 16, 18 gelagert sein, oder bei einem mehrfach unterteilten Gehäuseinnenraum mit einer auswechselbaren Zwischenwand 62 derart kombiniert sein kann, dass sie mittels einer quer durch die Achsmitte verlaufenden Doppelnut 64 in einen Schlitz 66 der Zwischenwand 62 eingeschoben werden kann.

Desweiteren kann die Lagerwelle bzw. Lagerachse 60 mit der oder den auswechselbaren Zwischenwänden 62 fest verbunden sein und mit ihnen eine im wesentlichen steife Baueinheit bilden.

Wie aus Fig. 4 ersichtlich ist, sind das nach oben abgewinkelte Bodenteilstück 22' sowie das untere Teilstück 26' der vorderen Gehäusestirnwand 26 vorzugsweise unter gleichen Winkeln schräg gestellt, so dass die wenigstens eine in entsprechenden Führungsnuten der vorderen Gehäusestirnwand 26 sowie der Gehäuserückwand 19 auswechselbar geführte Zwischenwand 62 auch seitenverkehrt in das Gehäuse eingesetzt werden kann. Diese Konstruktion erlaubt es desweiteren durch die Anordnung einer oder mehrerer Zwischenwände 62 jeweils mehrere Rollen auch unterschiedlicher Breite einzusetzen.

## Patentansprüche

1. Spenderbox zur Entnahme insbesondere vorproportionierter Teilstücke von ein- oder mehrbahnig zu einer Rolle aufgewickeltem handförmigem Packungsgut, wie Verbandstoffe und/oder Verbandsmaterial, mit einem dieses aufnehmenden, verschliessbaren, quaderförmigen Gehäuse (10) mit einer an dessen einer Gehäuseschmalseite (26) vorgesehen, mittels einer um eine Schwenkachse verschwenkbaren Verschlussklappe (38) verschliessbaren,eine der Bahnbreite des Packungsgutes entsprechenden Breite aufweisenden schlitzförmigen Entnahmeöffnung (24),
   **dadurch gekennzeichnet,**
   dass die Höhe der Entnahmeöffnung (24) geringfügig grösser als die Dicke des bahnförmigen Packungsgutes (58) ist und dass die untere Schlitzlängswand (28) mit ihrer vorderen Kante ein durchgehendes Auflager (32) für einen von der Schwenkachse entfernt liegenden Abschnitt der sich selbsttätig in ihre Schliesstellung zurückstellende Verschlussklappe (38; 42; 52) bildet.

2. Spenderbox nach Anspruch 1, dadurch gekennzeichnet, dass die Verschlussklappe (38; 42; 52) in der Schliesstellung über das Auflager (32) vorsteht oder dieses mit einem abgewinkelten, vorderen Klappenrandstück (38′) übergreift.

3. Spenderbox nach Anspruch 1 oder 2, dadurch gekennzeichnet, dass die das Auflager bildende Vorderkante des unteren Wandteils (28) durch einen nach oben gerichteten Auflagesteg (32) gebildet ist.

4. Spenderbox nach Anspruch 3, dadurch gekennzeichnet, dass sich der Auflagesteg (32) nach oben insbesondere spitzkeilförmig verjüngt.

5. Spenderbox nach einem der vorhergehenden Ansprüche, dadurch gekennzeichnet, dass die Entnahmeöffnung (24) sich am Vorderende eines unteren und eines zu diesem parallelen oberen ebenen Wandteils (28; 30) sowie zwischen Seitenwangen befindet, die sich in Ausziehrichtung des Packungsgutes (58) erstrecken, wobei der untere Wandteil (28) über die Vorderkante (34) des oberen Wandteils (30) vorsteht und dessen Vorderkante das stegartige Auflager (32) bildet.

6. Spenderbox nach Anspruch 5, dadurch gekennzeichnet, dass die Vorderkante (34) des oberen Wandteils (30) in der Mitte eine Aussparung (36) aufweist und dass die Schwenkachse der Verschlussklappe (38) oberhalb des oberen Wandteils (30) und hinter dessen Vorderkante (34) vorgesehen ist.

7. Spenderbox nach Anspruch 5, dadurch gekennzeichnet, dass die Verschlussklappe (52) den vorderen Abschnitt des oberen Wandteils (30) bildet.

8. Spenderbox nach Anspruch 7, dadurch gekennzeichnet, dass die Verschlussklappe (52) an ihrer in der Schliesstellung dem unteren Wandteil (28) zugekehrten Seite im Abstand zu ihrer Vorderkante einen zu dieser insbesondere parallel verlaufenden Steg (56) aufweist,der in der Schliesstellung der Verschlussklappe (52) zumindest auf dem Packungsgut (58) aufruht.

9. Spenderbox nach Anspruch 8, dadurch gekennzeichnet, dass in der Schliesstellung der Verschlussklappe (52) das Packungsgut (58) zwischen unterem Wandteil (28) und Steg (56) fixiert ist.

10. Spenderbox nach einem der vorhergehenden Ansprüche, dadurch gekennzeichnet, dass die das Auflager (32) bildende Vorderkante des unteren Wandteils (28) im wesentlichen mit den vorderen Gehäuseseitenkanten abschliesst.

11. Spenderbox nach einem der vorhergehenden Ansprüche, dadurch gekennzeichnet, dass der untere Wandteil (28) eine Verlängerung des in Entnahmerichtung abfallenden Gehäusebodens (22) bildet.

12. Spenderbox nach Anspruch 11,dadurch gekennzeichnet, dass die Gehäuseseitenwände (16; 18) und ggf. auch die Gehäuserückwand (19) über den Gehäuseboden (22) nach unten vorstehen.

13. Spenderbox nach Anspruch 12,dadurch gekennzeichnet, dass in nach unten über den Gehäuseboden (22) vorstehenden Teil der Gehäuseseitenwände (16; 18) und/oder in der Gehäuserückwand (19) jeweils eine sich von unten nach oben erstreckende, insbesondere schlitzförmige Ausnehmung (50) angeordnet ist.

14. Spenderbox nach Anspruch 12 oder 13, dadurch gekennzeichnet, dass zumindest die Gehäuseseitenwände (16; 18) im Bereich der Entnahmeöffnung (24) ihre grösste Höhe aufweisen.

15. Spenderbox nach einem der vorhergehenden Ansprüche, dadurch gekennzeichnet, dass in das Gehäuse (10) mindestens zwei Rollen an Packungsgut in einander benachbarte, getrennte Räume einsetzbar sind, die in eine gemeinsame Entnahmeöffnung ausmünden.

16. Spenderbox nach Anspruch 15,dadurch gekennzeichnet, dass einander benachbarte Gehäuseräume durch jeweils eine aus dem Gehäuse (10) herausnehmbare Zwischenwand (62) von einander getrennt sind.

17. Spenderbox nach einem der vorhergehenden Ansprüche, dadurch gekennzeichnet, dass das aufgewickelte Packungsgut (58) im Gehäuse (10) auf eine Achse (60) aufsteckbar ist.

**Claims**

1. Dispenser box for the removal of tape-like packing material such as bandages and/or bandaging material, in particular preproportioned sections, wound in single or multiple widths on a reel, comprising a closable housing (10) of right parallelepiped configuration receiving said packing material and having on its one narrow housing side (26) a slit-shaped removal opening (24) closable by a closure flap (38) pivotable about a pivot axis and having a width corresponding to the width of said packing material, characterized in that the height of said removal opening (24) is slightly greater than the thickness of said tape-like packing material (58), and in that the bottom longitudinal wall (28) of said slit forms with its front edge a continuous abutment (32) for a section of said closure flap (38; 42; 52) which returns automatically to its closed position, said section being remote from said pivot axis.

2. Dispenser box as defined in claim 1, characterized in that in said closed position, said closure flap (38; 42; 52) protrudes beyond said abutment (32) or engages over it with a front, angular flap edge section (38').

3. Dispenser box as defined in claim 1 or 2, characterized in that the front edge of said bottom wall part (28) forming said abutment is formed by an upwardly directed supporting ridge (32).

4. Dispenser box as defined in claim 3, characterized in that said supporting ridge (32) tapers upwards, in particular in wedge-shaped configuration, to a point.

5. Dispenser box as defined in one of the preceding claims, characterized in that said removal opening (24) is located at the front end of a bottom wall part (28) and a top flat wall part (30) parallel to the latter and between side cheeks extending in the pull-out direction of said packing material (58), said bottom wall part (28) protruding beyond the front edge (34) of said top wall part (30) and its front edge forming said ridge-like abutment (32).

6. Dispenser box as defined in claim 5, characterized in that said front edge (34) of said top wall part (30) has a recess (36) at the center thereof, and in that said pivot axis of said closure flap (38) is provided above said top wall part (30) and behind its front edge (34).

7. Dispenser box as defined in claim 5, characterized in that said closure flap (52) forms the front section of said top wall part (30).

8. Dispenser box as defined in claim 7, characterized in that said closure flap (52) has on its side which in the closed position faces said bottom wall part (28) in spaced relation to its front edge a ridge (56) which, in particular, extends parallel to said front edge and in the closed position of said closure flap (52) at least rests on said packing material (58).

9. Dispenser box as defined in claim 8, characterized in that in the closed position of said closure flap (52), said packing material (58) is fixed between bottom wall part (28) and ridge (56).

10. Dispenser box as defined in one of the preceding claims, characterized in that the front edge of said bottom wall part (28) forming said abutment (32) terminates essentially with said front side edges of said housing.

11. Dispenser box as defined in one of the preceding claims, characterized in that said bottom wall part (28) forms an extension of the housing bottom (22) which extends downwards in the removal direction.

12. Dispenser box as defined in claim 11, characterized in that said housing side walls (16; 18) and possibly also said housing rear wall (19) protrude downwards beyond said housing bottom (22).

13. Dispenser box as defined in claim 12, characterized in that a recess (50) extending from the bottom upwards, in particular, of slit-shaped configuration, is arranged in the part of said housing side walls (16; 18) protruding downwards beyond said housing bottom (22) and/or in said housing rear wall (19).

14. Dispenser box as defined in claim 12 or 13, characterized in that at least said housing side walls (16; 18) are highest in the region of said removal opening (24).

15. Dispenser box as defined in one of the preceding claims, characterized in that at least two reels of packing material are insertable in said housing (10) in adjacent, separate compartments which open into a common removal opening.

16. Dispenser box as defined in claim 15, characterized in that adjacent housing compartments are separated from one another by an intermediate wall (62) removable from said housing (10).

17. Dispenser box as defined in one of the preceding claims, characterized in that said wound-up packing material (58) is positionable on an axle (60) in said housing (10).

**Revendications**

1. Boîte dispensatrice permettant de prélever des portions, notamment prédosées, d'un produit en forme de bande, constitué d'un ou plusieurs rubans, tel que de l'étoffe et/ou de la matière pour pansement, enroulé sur un rouleau, ladite boîte comportant une enceinte parallélépipédique (10) pouvant être fermée, recevant ledit produit, et une ouverture de prélèvement (24) en forme de fente, laquelle présente une largeur correspondant à la largeur de la bande de produit, peut être fermée au moyen d'un volet obturateur (38) capable de pivoter autour d'un axe de pivotement et est prévue sur un premier côté étroit (26) de ladite enceinte, caractérisée
par le fait que la hauteur de l'ouverture de prélèvement (24) est légèrement plus grande que l'épaisseur du produit en bande (58), et par le fait que la paroi longitudinale inférieure de la fente (28) constitue, par son bord antérieur, un appui continu (32) pour une portion du volet obturateur (38; 42; 52) éloignée de l'axe de pivotement dudit volet obturateur, lequel revient automatiquement à sa position fermée.

2. Boîte dispensatrice selon revendication 1, caractérisée par le fait qu'à la position fermée, le volet obturateur (38; 42; 52) déborde au-delà de l'appui (32), ou chevauche celui-ci par un bord antérieur coudé (38').

3. Boîte dispensatrice selon revendication 1 ou 2, caractérisée par le fait que l'arête antérieure de la partie de paroi inférieure (28), formant l'appui, est constituée par une nervure d'appui (32) dirigée vers le haut.

4. Boîte dispensatrice selon revendication 3, caractérisée par le fait que la nervure d'appui (32) s'amincit vers le haut, notamment en forme de coin à profil aigu.

5. Boîte dispensatrice selon l'une des revendications précédentes, caractérisée par le fait que l'ouverture de prélèvement (24) se trouve à l'extrémité antérieure d'une partie de paroi plane inférieure (28) et d'une partie de paroi plane supérieure (30), parallèle à la précédente, ainsi qu'entre des joues latérales qui s'étendent dans la direction d'extraction du produit (58), la partie de paroi inférieure (28) formant, par son arête antérieure, l'appui du genre nervure (32) et débordant au-delà de l'arête antérieure (34) de la partie de paroi supérieure (30).

6. Boîte dispensatrice selon revendication 5, caractérisée par le fait que l'arête antérieure (34) de la partie de paroi supérieure (30) présente en son milieu une échancrure (36), et par le fait que l'axe de pivotement du volet obturateur (38) est prévu au-dessus de la partie de paroi supérieure (30) et derrière l'arête antérieure (34) de celle-ci.

7. Boîte dispensatrice selon revendication 5, caractérisée par le fait que le volet obturateur (52) forme la portion antérieure de la partie de paroi supérieure (30).

8. Boîte dispensatrice selon revendication 7, caractérisée par le fait qu'en son côté qui, à la position fermée, est tourné vers la partie de paroi inférieure (28), le volet obturateur (52) présente, à distance de son arête antérieure, une nervure (56) s'étendant notamment parallèlement à celle-ci et reposant au moins sur le produit lorsque le volet obturateur (52) est en position fermée.

9. Boîte dispensatrice selon revendication 8, caractérisée par le fait que, lorsque le volet obturateur (52) est en position fermée, le produit (58) est fixé entre nervure (56) et partie de paroi inférieure (28).

10. Boîte dispensatrice selon l'une des revendications précédentes, caractérisée par le fait que l'arête antérieure de la partie de paroi inférieure (28), formant l'appui (32), se termine sensiblement avec les arêtes latérales antérieures de l'enceinte.

11. Boîte dispensatrice selon l'une des revendications précédentes, caractérisée par le fait que la partie de paroi intérieure (28) forme un prolongement du fond (22) de l'enceinte, lequel est en déclivité dans la direction de prélèvement.

12. Boîte dispensatrice selon revendication 11, caractérisée par le fait que les parois latérales (16; 18) de l'enceinte et éventuellement aussi la paroi arrière (19) de celle-ci débordent vers le bas, au-delà du fond (22) de l'enceinte.

**13.** Boîte dispensatrice selon revendication 12, caractérisée par le fait qu'un évidement (50), notamment en forme de fente, s'étendant du bas vers le haut; est aménagé dans la partie des parois latérales (16; 18) débordant vers le bas au-delà du fond (22) de l'enceinte, et/ou dans la paroi arrière (19) de l'enceinte.

**14.** Boîte dispensatrice selon revendication 12 ou 13, caractérisée par le fait qu'au moins les parois latérales (16; 18) de l'enceinte présentent leur hauteur maximale dans la région de l'ouverture de prélèvement (24).

**15.** Boîte dispensatrice selon l'une des revendications précédentes, caractérisée par le fait qu'au moins deux rouleaux de produit peuvent être placés dans l'enceinte (10), dans des volumes voisins séparés qui débouchent dans une ouverture de prélèvement commune.

**16.** Boîte dispensatrice selon revendication 15, caractérisée par le fait que les volumes voisins que comporte l'enceinte sont séparés l'un de l'autre par une cloison (62) pouvant être enlevée de l'enceinte (10).

**17.** Boîte dispensatrice selon l'une des revendications précédentes, caractérisée par le fait que le produit enroulé (58) dans l'enceinte (10) peut être emboîté sur un axe (60).

# Fig. 2

# Fig. 1

# Fig. 3a

# Fig. 3

Fig.5

Fig.4

Fig.6